# EUROPEAN PATENT SPECIFICATION

(11) **EP 0 257 915 B1**
(45) Date of publication and mention of the grant of the patent: **10.03.1993**
(21) Application number: 87307115.3
(22) Date of filing: 11.08.1987
(51) Int. Cl.: A61K 9/50, A61K 9/72

(54) **Pharmaceutical formulations comprising microcapsules**
Mikrokapseln enthaltende pharmazeutische Zusammensetzungen
Formulations pharmaceutiques contenant des microcapsules

(30) Priority: 11.08.1986 GB 8619519; 05.01.1987 GB 8700063
(43) Date of publication of application: 02.03.1988
(73) Proprietor: INNOVATA BIOMED LIMITED, Edinburgh EH1 3PT, (GB)
(72) Inventor: Boyes, Robert Nichol, St. Albans Hertfordshire AL1 4ET (CA); Tice, Thomas Robert, Birmingham Alabama 35255-5305 (US); Gilley, Richard Mac, Birmingham Alabama 35255-5305 (US); Pledger, Kenneth Lawrence, Birmingham Alabama 35255-5305 (US)
(74) Representative: Woods, Geoffrey Corlett

(56) References cited:
- EP-A- 0 038 979
- EP-A- 0 140 085
- EP-A- 0 158 441
- CHEMICAL ABSTRACTS, vol. 71, no. 6, 11th August 1969, page 19, abstract no. 22526u, Columbus, Ohio, US; M. KOISHI et al.: "Microcapsules. II. Preparation of polyphthalamide microcapsules", & CHEM. PHARM. BULL. (TOKYO) 1969, 17(4), 804-9
- Kirk-Othmer, Encyclopedia of Chem. Technol., 3rd ed. 1981, vol. 15, pp. 470, 476-477, 479-481

## Description

This invention relates to controlling the release of drugs from pharmaceutical formulations.

Currently available treatments for asthma and bronchitis, although generally effective, are limited by the necessity for frequent drug administration and/or the possibility of unpleasant or debilitating side effects. It has long been established that direct application of bronchodilating drugs to the lungs by inhalation provides rapid relief for asthmatic and bronchitic symptoms. However, the very rapid systemic absorption of drugs when administered by this route results in a relatively short duration of the desired clinical effect. Consequently this route of administration has not been as acceptable for the prophylactic administration of bronchodilating drugs in an attempt to prevent acute asthmatic attacks. Certain oral dosage forms are available for this latter purpose. However, they are not administered directly to the site of action. The dosages required are therefore very much higher and the incidence of unpleasant side effects is much greater.

The respiratory tract may also be used as a means of drug administration in circumstances where other routes are inappropriate or inconvenient. Certainly anaesthetic agents are administered by this route for their systemic activity, and there is evidence in the literature that peptides such as insulin can be absorbed systematically from the lungs.

The process of microencapsulation of drugs in various polymeric materials is a well known means of producing controlled release drug delivery systems. EP-A-0038979, for example, describes the preparation of ethylcellulose microcapsules having a size less than 1000 µm, typically 100-450 µm, for the delivery of drugs. The disclosed process involves employing an organosilicone polymer, optionally together with a surfactant, to increase the viscoelasticity and wettability of the ethylcellulose gel. This measure serves to improve the quality of the resulting microcapsules by promoting the formation of a compact and complete ethylcellulose wall structure. The organosilicone polymer and optional surfactant are washed off the microcapsules at the end of the process.

Factors controlling the release of drugs from known microcapsules have been well described in the literature. In summary the critical factors are:
1. the method of encapsulation and therefore the physical nature of the microcapsule;
2. the specific polymeric material chosen to form the walls of the microcapsule;
3. the physical state of the polymeric material in the microcapsule, i.e. the degree of crystallinity; and
4. the solubility and diffusion characteristics of the salt form of the drug chosen.

Children and the elderly have difficulty in swallowing conventional tablets and capsules. Unfortunately there have been many technical difficulties in producing controlled release drug delivery systems which could be administered in syrup or liquid form. The greatest technical difficulties are, on the one hand, of keeping the active ingredient largely out of aqueous solution during storage, while at the same time allowing this active ingredient to dissolve slowly once the product has been administered to a patient.

We have now produced microencapsulated drug particles typically in sizes ranging in excess of 1 µm. The drugs employed have been incorporated in various polymeric wall forming materials. It was found that when these microcapsules were exposed to lipid-soluble surfactant or when such a surfactant was incorporated in the wall material of the microcapsule, the release of drug from the microcapsules was retarded. The rate of release of the drug could be controlled with respect to time.

Accordingly, the present invention provides pharmaceutical formulations suitable for inhalation, comprising:
(i) microcapsules having an average diameter of from 0.1 to 10 um which consist essentially of a biocompatible biodegradable polymeric wall material encapsulating a drug, and
(ii) a lipid-soluble surfactant which is mixed with the microcapsules or is incorporated within or coats the wall material of the microcapsules.

The invention further provides pharmaceutical compositions suitable for ingestion, comprising:
(i') microcapsules having an average diameter of from 0.1 to 20 µm, which consist essentially of a biocompatible polymer wall material encapsulating a drug, and
(ii) a lipid-soluble surfactant, which is mixed with the microcapsules or is incorporated within or coats the wall material of the microcapsules.

Such formulations can be simple mixtures of microcapsules encapsulating a drug and surfactant. Alternatively or additionally, microcapsules encapsulating drug may have been exposed to surfactant so that the surfactant forms a coating on the wall material of the microcapsules. Microcapsules having surfactant incorporated in their walls may also be prepared by dissolving the polymeric material for the walls in a solvent, dispersing surfactant and drug in the polymer solution, evaporating off the solvent and obtaining the particles thus formed with the desired average diameter. A pharmaceutically acceptable carrier or diluent may be provided in the formulation.

The formulation may be presented in a form suitable for inhalation. For this purpose, it may take the form of an aerosol in which the microcapsules are suspended in the propellant for the aerosol or it may be in dry powder form. Use of the present system for local application of drugs to the lungs extends beyond bronchodilating agents. Other drugs important in the treatment of asthma may be administered in this way including corticosteroids, disodium cromoglycate antihistamines and leukotriene antagonists. Furthermore, slow release of antibiotic and antiviral materials, chemotherapeutic agents for cancer treatment in the lung may be achieved.

Alternatively, an orally administrable preparation may be formulated. A preferred such oral formulation is prepared by suspending the microcapsules incorporating the surfactant in their walls in a pharmaceutically acceptable water-immiscible oil and emulsifying the oil suspension of microcapsules in an aqueous medium.

By mixing the lipid-soluble surfactant with the microcapsules or incorporating the surfactant in the walls of the microcapsules, the release of drug from the microcapsules can be Controlled. In its turn, this controls the degree and duration of the pharmacological effect obtained. As far as the preferred liquid oral dosage form is concerned, a drug release delivery system can be produced with is stable in the aqueous form but which releases drug in vivo at rates similar to that produced by various conventional long acting formulations. Further, the system is easier to administer to children and older patients. Protection of drugs by way of microencapsulation in the system can improve their stability in aqueous media. The flavour and palatability of liquid formulations may be improved.

The drug encapsulated in the microcapsules may be any agent which exhibits a pharmacological effect. The drug may be selected from antibiotics such as ampicillin or penicillin V, cardiovascular drugs such as betablockers, calcium antagonists or nitrates such as isosorbide dinitrate or isosorbide mononitrate, a drug for cough and cold preparations such as dextromethorphan or diphenhydramine, peptide drugs such as insulin or human growth hormone, other naturally occurring agents and derivatives such as prostaglandins, anti-viral agents and anti-convulsants such as phenytoin and sodium valproate.

Preferably, the drug is a bronchodilating agent. Suitable bronchodilator compounds include beta-receptor agonists and, more particularly, beta-adrenergic agonist agents such as salbutamol (2-tert.butylamino-1-(4-hydroxy-3-hydroxymethyl phenyl)ethanol, normally presented as its sulphate) and terbutaline (2-tert.butylamino-1-(3,5-dihydroxyphenyl)ethanol, normally presented as its sulphate). Other bronchodilating agents which may be employed are xanthines such as theophylline, anti-cholinergic agents such as ipatropium bromide and the like, calcium antagonists such as nifedipine and biological agents such as leukotrienes and derivatives thereof.

The amount of drug incorporated in the microparticles usually ranges from less than 1% to as high as 95% by weight, preferably from 1 to 80% by weight. Two or more drugs may be encapsulated in the microcapsules. In such an event, the drugs must be inert with respect to each other.

The polymeric wall material of the microcapsules must be biocompatible. A biocompatible polymeric material is a polymeric material which is not toxic to the human body, is not carcinogenic and should not induce inflammation in body tissues. Preferably, the wall material is biodegradable in the sense that it should degrade by bodily processes to products readily disposable by the body and should not accumulate in the body. For microcapsules for inhalation, therefore, the wall material should be biodegradable. Suitable examples of polymeric materials include poly(glycolic acid), poly-d,1-lactic acid copolymers thereof, copolyoxalates, polycaprolactone, and poly(lactic acid-caprolactone). For oral preparations, cellulose derivatives such as ethyl cellulose may be employed.

The molecular weight of the polymeric material for the walls of the microcapsules should be high enough so that it forms satisfactory polymer coatings. Usually a satisfactory molecular weight is greater than 10,000 daltons. The molecular weight of a polymer is also important from the point of view that molecular weight influences the biodegradation rate of the polymer. By an appropriate selection of polymeric materials a formulation can be made such that the resulting microcapsules remain intact until all of the drug is released and then degrade.

The microcapsules may be prepared in any suitable fashion. They may be prepared by spray-drying. In this technique, the polymeric material for the walls of the microcapsules is dissolved in a solvent such as methylene chloride. An appropriate amount of surfactant, if desired, and drug, depending on the desired core load of the microcapsules, is dispersed in the polymer solution. This dispersion is then spray-dried. An inert gas such as nitrogen is typically forced through the nozzle of the spray-dryer with the dispersion to cause an aerosol of microcapsules to be formed initially. The microcapsules are then collected.

Alternatively, the microcapsules may be prepared by:
(a) dissolving or dispersing a drug in a solvent, typically methylene chloride, and dissolving a biocompatible and biodegradable wall forming material in the solvent;
(b) dispersing the solvent containing the drug and wall forming material in a continuous phase processing medium such as a 1 to 10% by weight polyvinyl alcohol in water mixture;
(c) evaporating a portion of the solvent from the dispersion of step (b), thereby forming microcapsules containing the drug in suspension; and
(d) extracting the remainder of the solvent from the microcapsules.

The microparticle product is usually made up of particles of a spherical shape although sometimes the microcapsules may be irregularly shaped. Microcapsules for inhalation have an average diameter of 0.1 to 10 µm, preferably 1 to 5 µm. Microcapsules for ingestion have an average diameter of from 0.1 to 20 µm, preferably from 1 to 20 µm.

The microcapsules incorporate a lipid-soluble surfactant as a drug release controlling agent. The surfactant is typically in liquid form. It is preferably a sorbitan fatty acid ester such as sorbitan trioleate, sorbitan monolaurate, sorbitan monooleate, sorbitan monopalmitate and sorbitan monostearate. Alternatively, the surfactant may be a polyoxamer or a surface active fatty acid such as oleic acid.

Where the formulation is a simple mixture of microcapsules and surfactant, the surfactant is typically present in an amount up to twice the weight, preferably up to the weight, of microcapsules. However, in the case of an aerosol the amount of surfactant is generally rather less than this. Surfactant can be incorporated in the walls of the microcapsules in an amount of up to 25% by weight of the microcapsules. Preferably, the amount is at least 1% by weight. Where more than 10% by weight is used, little useful additional effect is achieved. When microcapsules are exposed to surfactant to enable the surfactant to become coated on the microcapsule walls, typically the amount of surfactant to which the microcapsules are exposed is up to ten times, for example up to twice or more, the weight, preferably up to the weight, of the microcapsules.

Formulations according to the invention may be presented in a form suitable for inhalation. For this purpose, the formulations are generally loaded in an aerosol canister or, in dry powder form, in an inhaler. In an aerosol, the microcapsules are suspended in the propellant for the aerosol. In a dry powder inhaler, the microcapsules may be mixed with a diluent such as lactose. Typically, such microcapsules have had surfactant incorporated in their walls during the encapsulation procedure. In both ways, a fine suspension of microcapsules may be taken into the lungs by a patient. Typically, the aerosol canister or inhaler allows a metered dose of a drug such as a bronchodilating agent to be inhaled. This is generally achieved by the provision of a valve which meters the amount of formulation, fluid in the case of an aerosol, discharged with each actuation.

The microcapsules may be contacted with the surfactant prior to loading in an aerosol. Preferably, however, the surfactant is provided with the microcapsules and the propellant for the aerosol in the aerosol cannister. In such an instance, the surfactant may be provided in an amount ranging down to 10% of the weight of the microcapsules. The concentration of surfactant in the mixture in the aerosol cannister is typically no more than 5% by weight, preferably from 0.01 to 1% by weight. In the case of microcapsules for a dry powder inhaler, the surfactant is incorporated in the microcapsule walls prior to loading the inhaler with the microcapsules.

Formulations according to the invention may alternatively be ingested orally. The microcapsules are exposed to surfactant and suspended in a pharmaceutically acceptable water-immiscible oil. The oil is typically liquid, a fixed oil such as a fatty acid ester of glycerol. For example, the oil may be cotton seed oil or ethyl oleate. This suspension is then emulsified in an aqueous medium. By altering the drug loading in the microcapsules and by varying the relative concentrations of surfactants and oil, it is possible to alter the amount of drug immediately available in the aqueous phase of the mixture as well as control the rate of release of drug from the mixture into systems which mimic oral absorption.

The formulations are administered to a patient by inhalation or orally. A therapeutically effective amount is taken by a patient. Dosages depend upon the condition being treated, the stage the condition has reached, the patient under treatment and the drug being administered. Typically, however, one or two doses per day of a formulation of the invention may be given to a patient.

In a preferred embodiment where a bronchodilating agent is encapsulated for inhalation, asthma, bronchitis and other diseases of the respiratory system may be treated. The amount of formulation administered depends on the particular disease or disorder being treated and the type of bronchodilating agent being administered. Generally, however, the formulations may be inhaled only once or twice during a day. Thus, a formulation may be inhaled on two occasions per day, with an eight to twelve hour gap before the second inhalation, in an amount sufficient to deliver from 50 µg to 2 mg, more preferably 100 to 500 µg, of bronchodilating agent to the lungs per occasion. The dosage for salbutamol, for example, may be two inhalations of 100 µg each. This contrasts with the recommended dose for conventional salbutamol aerosol inhalations of 1 to 2 inhalations, each of 100 µg, every three to four hours up to a maximum of eight per twenty hours (Martindale, The Extra Pharmacopoeia, 28th edition, 1982). The dosage for terbutaline may be two inhalations each of 250 µg.

The following Examples 1 to 6 illustrate the invention. In the accompanying Figures:
Figure 1 shows the results of the assays of the supernatant for terbutaline sulphate in Example 2; and
Figure 2 shows the results of the dissolution test in Example 2.

### Reference Example: Preparation of terbutaline microcapsules

Terbutaline sulphate microcapsules were prepared using a Buchi 190 Mini spray-dryer equipped with a 0.7-mm spray nozzle (Brinkmann Instruments Co, Westbury, N.Y.). The spray-drying procedure was conducted as follows:
A 1.25 wt % polylactide-glycolide copolymer (DL-PLG) solution was prepared using methylene chloride as the solvent. After complete dissolution of the polymer, terbutaline sulphate was added to the polymer solution in an amount equalling the weight of DL-PLG. The terbutaline sulphate particles were then suspended by homogenization using a Brinkmann Polytron® homogenizer (Brinkmann Instruments Co, Westbury N.Y.). Immediately after the homogenization was completed, the drug/polymer mixture was pumped into the nozzle of the spray dryer at a flow rate of approximately 1 ml/min. To effect aerosolization, nitrogen was also directed through the nozzle. The nitrogen pressure was maintained at about 103 KPa (15 lbs/in²). The temperature of the spray-dryer chamber was kept at approximately 70°C. After all of the drug/polymer mixture was processed, the spray dryer was allowed to gradually cool to room temperature and the microcapsule product was collected.

### Example 1: Effect of sorbitan trioleate on the in vitro release of terbutaline from microcapsules

Solutions of sorbitan trioleate (Span 85) were prepared as follows. Into a 113g (4 oz) glass jar, 150 to 160g of Freon® was added via a gas cylinder. Next, an appropriate amount of Span® 85 was added to each jar. To prepare 1% Span 85 in Freon, approximately 1.5 g of Span 85 was weighed into a weigh boat and poured into the jar containing the Freon. To prepare more dilute surfactant solutions it was necessary to add the Span 85 surfactant via a pipette. It was determined that 10 drops of Span 85 weighed approximately 150 mg. Therefore 1 drop weighs approximately 15 mg.

| Solutions prepared | Span 85 added | Freon wt |
|---|---|---|
| 1% Span 85 | ∼1.5 g | 156.4 g |
| 0.25% | 25 drops | 158.0 g |
| 0.10% | 10 drops | 161.2 g |
| 0.01% | 1 drop | 150.7 g |

After the surfactant was added, the jars were capped with Teflon®-lined screw caps and shaken. The jars were stored at -70°C.

Microcapsules were weighed into plastic scintillation vials and different surfactant solutions were added to each vial individually. The vials were labelled accordingly, and a stir bar was added to each vial. The sample was allowed to stir uncovered for approximately 4 hours at room temperature. The samples were then placed under a hood and exposed to continuous air flow for 30 min to remove all traces of the Freon. The microcapsule/Span 85 mixture was then scraped from the vial.

An amount of microcapsules which contained approximately 15 mg of terbutaline sulphate was weighed out in triplicate and placed into small nylon pouches. These pouches consisted of 5 µm, nylon mesh (Small Parts Inc., Miami, Florida), and were 3.81 cm x 3.81 cm (1.5 in x 1.5 in). The pouches were formed by heat sealing the edges with a hot spatula blade. The microcapsules were sealed within the pouch by this method as well.

A nylon pouch containing the microcapsules was then placed in a 227 g (8 oz) glass jar, and 200 ml of deionized water was then added. Next, the glass jar was placed in a shakerbath maintained at 37°C and oscillating at a rate of about 120 cycles/min. Aliquots were then removed periodically from the receiving fluid and assayed spectrophotometrically at 227 nm to determine their terbutaline sulphate concentrations. The amounts of terbutaline sulphate released was then calculated by using the highest recorded absorbance to determine 100% release of drug from the microcapsules. After the maximum or 100% release was obtained, the % release was determined proportionally. To illustrate, the following data were obtained for Microcapsule Batch D743-021 after exposure to 0.10% Span 85 in Freon.

| Time | Abs. = 227 nm | Conc (µg/ml) | % Released |
|---|---|---|---|
| 5 min | 1.443 | 74.0 | 83.5 |
| 15 min | 1.650 | 85.4 | 96.4 |
| 30 min | 1.693 | 87.6 | 98.9 |
| 1 h | 1.711 | 88.6 | 100.0 |

Thus the maximum absorbance as determined spectrophotometrically at 227 nm was 88.6 µg/ml. This was determined via a standard curve which was done previously. After determining the maximum release at 1 h of 88.6 µg/ml, the percent release at the earlier times was calculated proportionally. For example, the release at 5 min was calculated as follows:

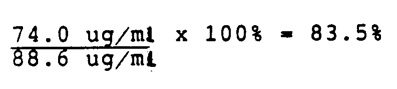

The results are shown in Table 1 below.

### Example 2: Liquid oral terbutaline formulation

Microcapsules of terbutaline sulphate were produced by spray drying according to the Reference Example. The particle sizes of the microcapsules were from 1 to 10 µm. The microcapsules were mixed with sorbitan trioleate surfactant; then mixed with the oil, ethyl oleate; and finally emulsified in water by first adding the non-ionic surfactant Cremophor® EL to the oil and microcapsule mixture and then adding quantities of water with vigorous mixing. The weight ratio of terbutaline microcapsules: sorbitan trioleate was about 1:5. The following quantitative formula was employed:

| | |
|---|---|
| Terbutaline microcapsules | 174 mg |
| Sorbitan trioleate | 1 ml |
| ethyl oleate | 5 ml |
| Cremophor EL | 5 ml |
| Water QS | 100 ml |

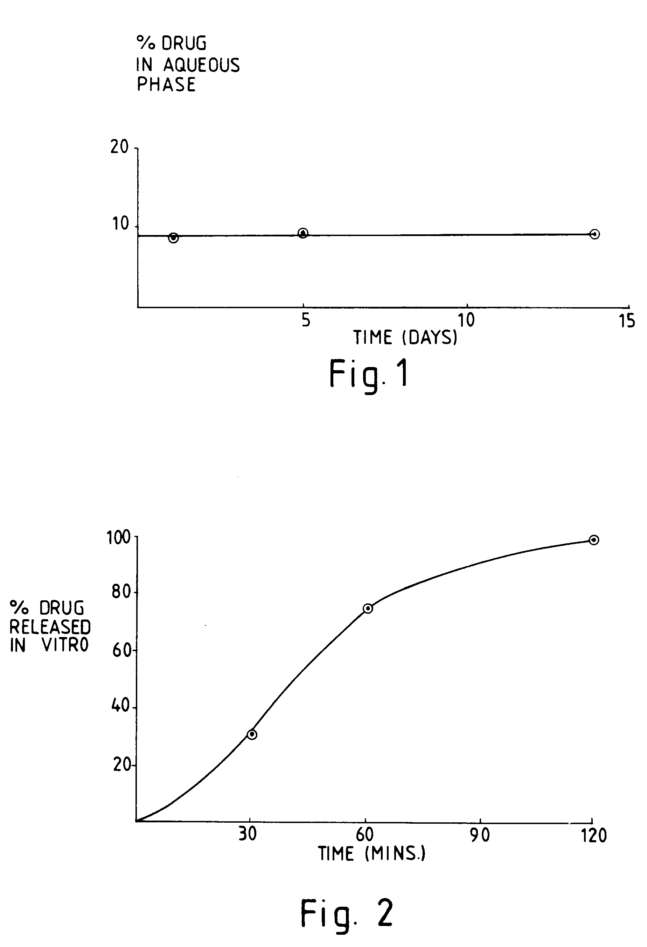

The aqueous supernatant was assayed for terbutaline content on the 1st, 5th and 14th day after production. Furthermore after the 14th day a 20ml sample of this mixture was exposed to the standard USP dissolution test run in pH 6.8 buffer at 37°C and a stirring frequency of 50 cycles/min. The results are shown in Figures 1 and 2.

In Figure 1, the data from the assays of supernatant when the mixture was stored for 14 days are presented. It is clear from this Figure that the concentration of terbutaline sulphate in the aqueous phase on the 1st day after production was 8.9% of the total terbutaline concentration and at 14 days the concentration was the same. By contrast the results in Figure 2 present data for the in vitro dissolution of this same liquid formulation. This pharmacopoeia test is the same as that recommended for many tablet and capsule long acting formulations. In Figure 2 it is quite apparent that when the mixture was exposed to this USP test, drug dissolved slowly but completely in the aqueous media over a 2 hour period.

### Example 3: Preparation of Aerosols containing Microcapsules

Experimental aerosols were prepared by adding an appropriate quantity of microcapsules of terbutaline sulphate to an empty aluminium canister. The microcapsules were produced by spray-drying according to the Reference Example. They were designated batch D743-055-1. Their core loading of terbutaline sulphate was 26.6% by weight. The amount of terbutaline sulphate released from the microcapsules, in the absence of surfactant, was measured in vitro by the procedure described in Example 1. The percentages by weight of terbutaline sulphate released after 5, 10 and 30 mins were 84.5%, 98.6% and 98.8%, respectively.

The remaining ingredients for the aerosol, suitably cooled, were added. A metered valve capable of delivering 25 ul of the mixture was crimped to the canister to provide a seal. The following aerosols were prepared:

| Aerosol No. 1 | |
|---|---|
| Microcapsule batch D743-055-1 | 200 mg |
| Sorbitan Trioleate | 140 mg |
| Trichlorofluoromethane (Propellant 11 BP 80) | 3.44 g |
| Dichlorotetrafluoroethane (Propellant 114 BP 80) | 3.44 g |
| Dichlorodifluoromethane (Propellant 12 BP 80) | 6.88 g |

| Aerosol No. 2 | |
|---|---|
| Microcapsule batch D743-055-1 | 200 mg |
| Trichlorofluoromethane (Propellant 11 BP 80) | 3.44 g |
| Dichlorotetrafluoroethane (Propellant 114 BP 80) | 3.44 g |
| Dichlorodifluoromethane (Propellant 12 BP 80) | 6.88 g |

### Example 4: In vivo studies involving terbutaline microcapsules

Aerosols prepared as described in Example 3 were tested in human volunteers. In one experiment, Aerosol No. 1 was employed. The physiological effects produced by this aerosol were compared to similar effects producted in the volunteer by a commercially available terbutaline aerosol. The human volunteer inhaled 4 puffs from the commercially available terbutaline inhaler (total inhaled dose approximately 1 mg terbutaline sulphate) and, on another occasion, inhaled 8 puffs from Aerosol No. 1 (inhaled dose approximately 1 mg terbutaline sulphate). Airway resistance was measured at regular intervals following inhalation of the drug substance, utilising a constant volume body plethysmograph and expressed as specific airway conductance (sGaw). This method has been described by others, for instance see J E Harvey and A E Tattersfield Thorax 1982; 37:280-287. The results of this experiment are given in Table 2 below. Time here and in Table 3 is in minutes.

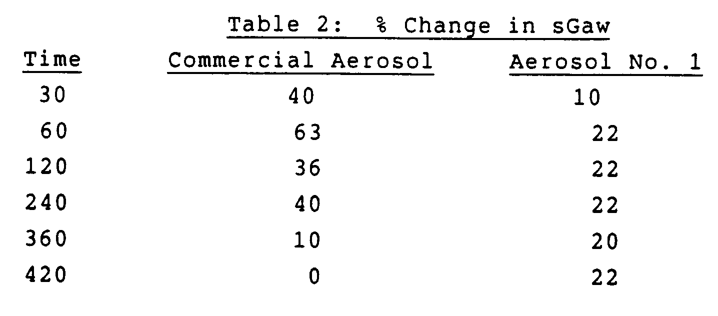

A second experiment was carried out using another human volunteer. Aerosol No. 2 of Example 3 was compared against a standard terbutaline aerosol. In this case the volunteer inhaled 4 puffs of the experimental formulation and 2 puffs from the standard inhaler, SGaw was again measured at regular intervals as described above. The results from this experiment are given in Table 3 below.

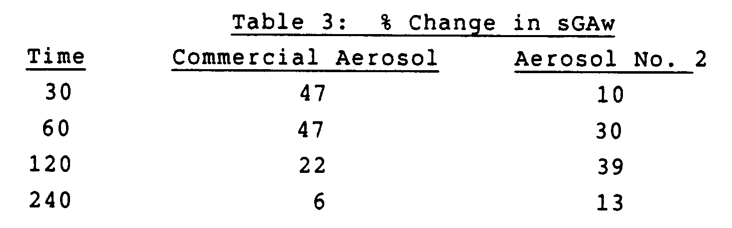

The data presented in Tables 2 and 3 clearly indicates that Aerosols Nos. 1 and 2, containing microencapsulated terbutaline, were capable of prolonging the effect of the drug on sGaw. Comparing the results from the first and second experiments indicates that the sorbitan trioleate component of the aerosol mixture also had a significant influence on the results. It is clear that the initial response to terbutaline from Aerosol No. 1 was substantially depressed and that the response was constant for up to six hours. By comparison, the maximum pharmacological effect for Aerosol No 2 was delayed by approximately one hour as a result of the microencapsulation of the drug. The response was not constant and was in steep decline by the time of four hours. These data clearly indicate that the surfactant prolongs the effect of the inhaled microcapsule formulation.

### Example 5: Preparation of Microcapsules incorporating Surfactant in their wall material

Salbutamol suphate microcapsules containing Span 85 were prepared using a Buchi 190 Mini spray-dryer equipped with a 0.7 mm spray nozzle (Brinkmann Instruments Co, Westbury, NY). The spray-drying procedure was conducted as follows.

A DL-PLG solution (0.80 wt %) was prepared using methylene chloride as the solvent. After complete dissolution of the polymer the span 85 was added. An amount of Span 85 was added such as to obtain a core loading of surfactant of 10% by weight. After the Span 85 was dispersed throughout the solution, the salbutamol sulphate was added. An amount of salbutamol sulphate was added such as to obtain a core loading of drug desired of 20.1% by weight. The salbutamol sulphate particles were then suspended by homogenisation using a Brinkmann Polytron homogeniser (Brinkmann Instruments Co, Westbury, NY). To perform this homogenisation procedure, the probe of the homogeniser was submerged in the polymer/drug mixture. The homogeniser was operated at a setting of approximately 6. The mixture was homogenised for three 30 second bursts, allowing 30 seconds between each burst.

Immediately after the homogenisation procedure, the polymer/drug mixture was placed on a stir plate and stirred vigorously to ensure that the salbutamol sulphate particles remained in suspension. A Gilson Minipulse 2 pump (Gilson Medical Electronics Inc, Middleton, WI) was then used to pump the mixture into the nozzle of the spray-dryer. A flow rate of approximately 1 ml/min was attained using size-14 Viton tubing (Cole-Parmer Instrument and Equipment Co, Chicago, IL). A small piece of aluminium foil was wrapped securely around the tubing and the mouth of the solvent jar to prevent loss of methylene chloride through evaporation.

To effect aerosolisation, nitrogen was directed through the nozzle of the spray-dryer. The nitrogen pressure was maintained at about 103 KPa (15 lb/in²) , as determined by the regulator on the nitrogen tank. The temperature of the spray-dryer's inlet chamber was maintained at about 50°C, while the collection chamber was kept at approximately 40°C. After all of the mixture was processed, the spray-dryer was allowed to gradually cool to room temperature and the microcapsule product was collected.

### Example 6: Measurement of In Vitro Release Rate from Microcapsules incorporating Surfactant in their wall material

In order to simulate release of drug in the lungs, a simple dissolution/diffusion apparatus was utilised. In this system, accurately weighed amounts of salbutamol-containing microcapsules prepared according to Example 5, of salbutamol-containing microcapsules prepared according to Example 5 but without Span 85 or pure salbutamol were placed inside a sealed piece of dialysis tubing. The pieces of tubing were then suspended in beakers containing 150 ml of pH 7.4 buffer. The buffer was stirred at a constant rate. Small samples were taken from the fluid in the beakers at regular intervals and assayed for active drug using the following procedure:
Samples of the buffer were injected directly on an HPLC fitted with a 2-Module with Novopak column and Guardpak pre-column, and a fluorescence detector set at 230 nm excitation with no emission filter. The solvent employed was a 4:96 acetonitrile/water with TEA adjusted to pH3 with orthophosphoric acid, and the flow rate was 2.5 ml per minute.
The release of salbutamol from the two different batches of microcapsules, one with and the other without surfactant, is demonstrated in Table 4 below. Clearly, the presence of the Span surfactant within the microcapsulses substantially delayed the release of the drug.

### Example 7

Salbutamol microcapsules were prepared using the reference method and an in vitro release study was carried out as described in Example 1 except that oleic acid was used as the surfactant instead of sorbitan trioleate. The results obtained are shown in Table 5 below.

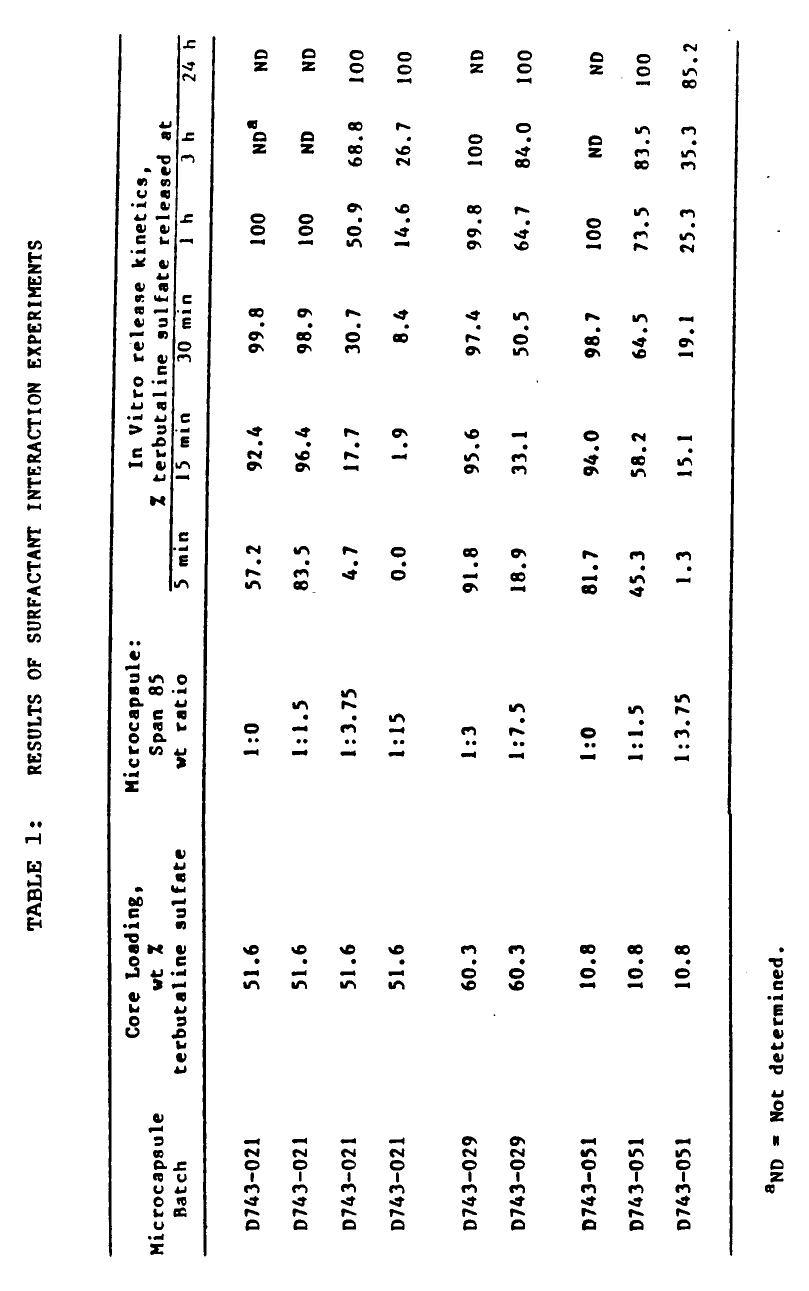

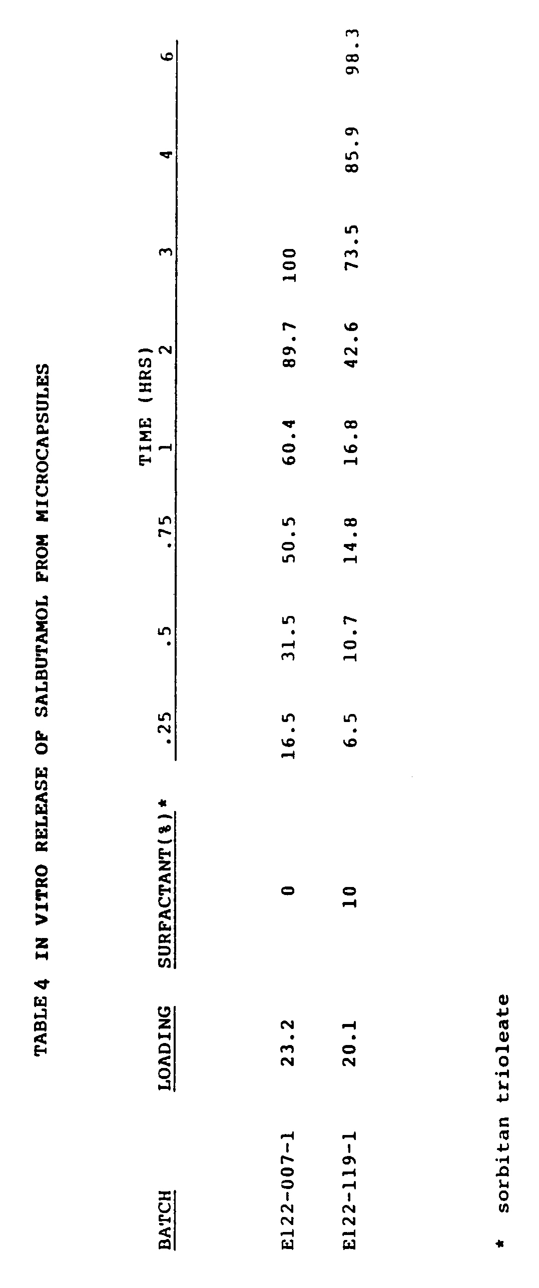

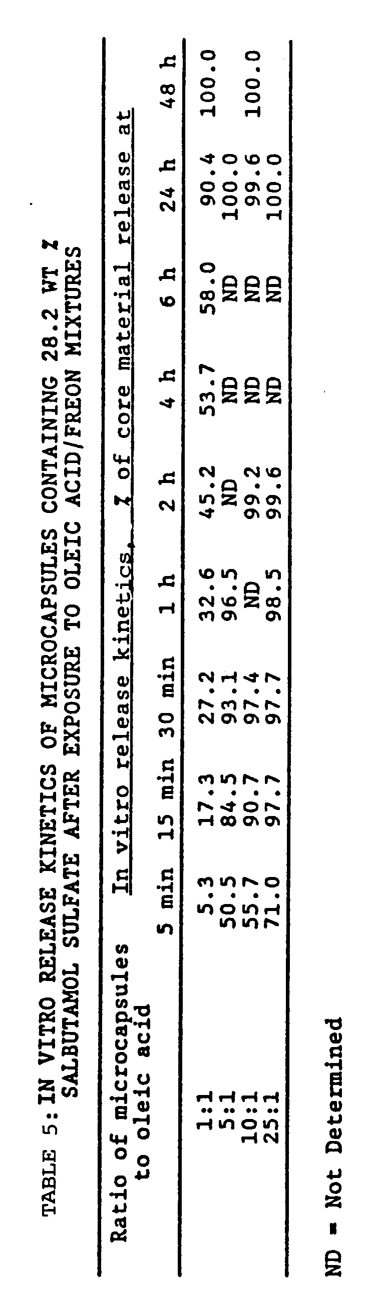

## Claims (Claims for the following Contracting State(s): AT, BE, CH, DE, FR, GB, IT, LI, LU, NL, SE)

1. A pharmaceutical formulation suitable for inhalation, comprising:
(i) microcapsules having an average diameter of from 0.1 to 10 µm which consist essentially of a biocompatible biodegradable polymeric wall material encapsulating a drug, and
(ii) a lipid-soluble surfactant which is mixed with the microcapsules or is incorporated within or coats the wall material of the microcapsules.

2. A formulation according to claim 1, which is in the form of an aerosol for inhalation.

3. A formulation according to claim 2, wherein the concentration of surfactant in the aerosol cannister is from 0.01 to 1% by weight.

4. A formulation according to claim 1, which is in the form of a dry powder for inhalation and wherein from 1 to 10% by weight of surfactant is incorporated in the walls of the microcapsules.

5. A pharmaceutical formulation suitable for ingestion, comprising:
(i') microcapsules having an average diameter of from 0.1 to 20 µm which consist essentially of a biocompatible polymer wall material encapsulating a drug, and
(ii) a lipid-soluble surfactant which is mixed with the microcapsules or is incorporated within or coats the wall material of the microcapsules.

6. A formulation according to claim 5, which is in the form of a composition suitable for oral administration wherein the microcapsules, which are coated with surfactant, are suspended in a pharmaceutically acceptable water-immiscible oil which is in turn emulsified in an aqueous medium.

7. A formulation according to any one of claims 1 to 6 wherein the surfactant is a sorbitan fatty acid ester.

8. A formulation according to claim 7, wherein the surfactant is sorbitan trioleate.

9. A formulation according to any one of claims 1 to 8 wherein the drug is an antibiotic, cardiovascular drug, a drug for a cough or cold preparation, a peptide drug, an anti-viral agent, an anti-convulsant or chemotherapeutic agent for cancer treatment.

10. A formulation according to any one of claims 1 to 8 wherein the drug is a bronchodilating agent or other anti-asthma drug selected from corticosteroids, disodium cromoglycate and antihistamines.

11. A formulation according to claim 10, wherein the bronchodilating agent is a beta-adrenergic agonist, a xanthine, an anti-cholinergic agent, a calcium antagonist or a leukotriene or derivative thereof.

12. A formulation according to claim 11, wherein the drug is salbutamol or terbutaline.

13. A formulation according to any one of claims 1 to 8 and 10 to 12, wherein microcapsules encapsulating a bronchodilating agent are mixed with a sorbitan fatty acid ester which is present in an amount up to the weight of the microcapsules.

14. A process for the preparation of a pharmaceutical formulation as defined in any one of claims 1 to 13 which process comprises mixing the microcapsules with the surfactant.

15. A process according to claim 14 which process comprises exposing the microcapsules encapsulating drug to surfactant so that the surfactant coats the wall material of the microcapsules.

16. A process for the preparation of a pharmaceutical formulation as defined in any one of claims 1 to 13 in which the surfactant is incorporated in the wall material of the microcapsules, which process comprises dissolving the polymeric material for the walls in a solvent, dispersing surfactant and drug in the polymer solution, evaporating off the solvent and obtaining the microcapsules thus formed with an average diameter of from 0.1 to 10 µm.

17. A process according to claim 16 for the preparation of a pharmaceutical formulation as defined in claim 5, further comprising suspending the microcapsules which have been exposed to surfactant in a pharmaceutically acceptable water-immiscible oil and emulsifying the resulting suspension in an aqueous medium.

## Claims (Claims for the following Contracting State(s): ES, GR)

1. A process for the preparation of a pharmaceutical formulation suitable for inhalation, comprising
(i) microcapsules having an average diameter of from 0.1 to 10 µm which consist essentially of a biocompatible biodegradable polymeric wall material encapsulating a drug, and
(ii) a lipid-soluble surfactant which is mixed with the microcapsules or is incorporated within or coats the wall material of the microcapsules; the process comprising mixing the microcapsules with the surfactant.

2. A process according to claim 1, wherein the formulation is in the form of an aerosol for inhalation.

3. A process according to claim 2, wherein the concentration of surfactant in the aerosol cannister is from 0.01 to 1% by weight.

4. A process according to claim 1, wherein the formulation is in the form of a dry powder for inhalation and wherein from 1 to 10% by weight of surfactant is incorporated in the walls of the microcapsules.

5. A process for the preparation of a pharmaceutical formulation suitable for ingestion, comprising:
(i') microcapsules having an average diameter of from 0.1 to 20 µm which consist essentially of a biocompatible polymer wall material encapsulating a drug, and
(ii) a lipid-soluble surfactant which is mixed with the microcapsules or is incorporated within or coats the wall material of the microcapsules; the process comprising mixing the microcapsules with the surfactant.

6. A process according to any one of claims 1 to 5 which process comprises exposing the microcapsules encapsulating drug to surfactant so that the surfactant coats the wall material of the microcapsules.

7. A process according to any one of claims 1 to 6, wherein the surfactant is incorporated in the wall material of the microcapsules, which process comprises dissolving the polymeric material for the walls in a solvent, dispersing surfactant and drug in the polymer solution, evaporating off the solvent and obtaining the mlcrocapsules thus formed.

8. A process according to any one of claims 5 to 7 wherein the formulation is in the form of a composition suitable for oral administration, the process comprising suspending the microcapsules which have been exposed to surfactant in a pharmaceutically acceptable water-immiscible oil and emulsifying the resulting suspension in an aqueous medium.

9. A process according to any one of claims 1 to 8 wherein the surfactant is a sorbitan fatty acid ester.

10. A process according to claim 9, wherein the surfactant is sorbitan trioleate.

11. A process according to any one of claims 1 to 10 wherein the drug is an antibiotic, cardiovascular drug, a drug for a cough or cold preparation, a peptide drug, an anti-viral agent, an anti-convulsant or chemotherapeutic agent for cancer treatment.

12. A process according to any one of claims 1 to 10 wherein the drug is a bronchodilating agent or other anti-asthma drug selected from corticosteroids, disodium cromoglycate and antihistamines.

13. A process according to claim 12, wherein the bronchodilating agent is a beta-adrenergic agonist, a xanthine, an anti-cholinergic agent, a calcium antagonist or a leukotriene or derivative thereof.

14. A process according to claim 13, wherein the drug is salbutamol or terbutaline.

15. A process according to any one of claims 1 to 10 and 12 to 14 which comprises mixing microcapsules encapsulating a bronchodilating agent with a sorbitan fatty acid ester in an amount up to the weight of the microcapsules.

## Patentansprüche (Patentansprüche für folgende(n) Vertragsstaat(en): AT, BE, CH, DE, FR, GB, IT, LI, LU, NL, SE)

1. Zur Inhalation geeignete pharmazeutische Formulierung, umfassend:
(i) Mikrokapseln, die einen mittleren Durchmesser von 0,1 bis 10 µm aufweisen, und die im wesentlichen aus einem biologisch kompatiblen, biologisch abbaubaren polymeren Wandmaterial bestehen, das einen Arzneistoff einkapselt und
(ii) ein lipidlösliches Surfactant, das mit den Mikrokapseln gemischt oder in das Wandmaterial der Mikrokapseln eingebaut ist oder dieses überzieht.

2. Formulierung nach Anspruch 1, die in Form eines Aerosols zur Inhalation vorliegt.

3. Formulierung nach Anspruch 2, wobei die Konzentration des Surfactants in dem Aerosolbehälter von 0,01 bis 1 Gew.-% beträgt.

4. Formulierung nach Anspruch 1, die in Form eines trockenen Pulvers zur Inhalation vorliegt und wobei von 1 bis 10 Gew.-% des Surfactants in die Wände der Mikrokapseln eingebaut ist.

5. Zum Einnehmen geeignete pharmazeutische Formulierung, umfassend:
(i') Mikrokapseln, die einen mittleren Durchmesser von 0,1 bis 20 µm aufweisen und die im wesentlichen aus einem biologisch kompatiblen, polymeren Wandmaterial bestehen, das einen Arzneistoff einkapselt und
(ii) ein lipidlösliches Surfactant, das mit den Mikrokapseln gemischt oder in das Wandmaterial der Mikrokapseln eingebaut ist oder dieses überzieht.

6. Formulierung nach Anspruch 5, die in Form einer zur oralen Verabreichung geeigneten Zusammensetzung vorliegt, wobei die Mikrokapseln, die mit Surfactant überzogen sind, in einem pharmazeutisch verträglichen, mit Wasser nichtmischbaren Öl suspendiert sind, das wiederum in einem wäßrigen Medium emulgiert ist.

7. Formulierung nach einem der Ansprüche 1 bis 6, wobei das Surfactant ein Sorbitanfettsäureester ist.

8. Formulierung nach Anspruch 7, wobei das Surfactant Sorbitantrioleat ist.

9. Formulierung nach einem der Ansprüche 1 bis 8, wobei der Arzneistoff ein Antibiotikum, ein kardiovaskulärer Arzneistoff, ein Arzneistoff für eine Zubereitung gegen Husten oder Erkältung, ein Peptidarzneistoff, ein Antivirusmittel, ein Antikonvulsivum oder ein chemotherapeutisches Mittel zur Behandlung von Krebs ist.

10. Formulierung nach einem der Ansprüche 1 bis 8, wobei der Arzneistoff ein Bronchodilatator oder ein anderes Antiasthmatikum ausgewählt aus Corticosteroiden, Dinatriumcromoglykat und Antihistaminika ist.

11. Formulierung nach Anspruch 10, wobei der Bronchodilatator ein beta-adrenerger Agonist, ein Xanthin, ein anticholinerges Mittel, ein Calciumantagonist oder ein Leukotrien oder ein Derivat davon ist.

12. Formulierung nach Anspruch 11, wobei der Arzneistoff Salbutamol oder Terbutalin ist.

13. Formulierung nach einem der Ansprüche 1 bis 8 und 10 bis 12, wobei Mikrokapseln, die einen Bronchodilatator einkapseln, mit einem Sorbitanfettsäureester gemischt werden, der in einer Menge bis zu dem Gewicht der Mikrokapseln vorliegt.

14. Verfahren zur Herstellung einer pharmazeutischen Formulierung nach einem der Ansprüche 1 bis 13, wobei das Verfahren das Mischen der Mikrokapseln mit dem Surfactant umfaßt.

15. Verfahren nach Anspruch 14, wobei das Verfahren umfaßt, daß die Mikrokapseln, die den Arzneistoff einkapseln, einem Surfactant ausgesetzt werden, so daß das Surfactant das Wandmaterial der Mikrokapseln überzieht.

16. Verfahren zur Herstellung einer pharmazeutischen Formulierung nach einem der Ansprüche 1 bis 13, in dem das Surfactant in das Wandmaterial der Mikrokapseln eingebaut ist, wobei das Verfahren das Auflösen des polymeren Materials für die Wände in einem Solvens, das Dispergieren des Surfactants und des Arzneistoffs in der Polymerlösung, das Verdampfen des Solvens und die Gewinnung der so gebildeten Mikrokapseln mit einem mittleren Durchmesser von 0,1 bis 10 µm umfaßt.

17. Verfahren nach Anspruch 16 zur Herstellung einer pharmazeutischen Formulierung nach Anspruch 5, darüber hinaus umfassend das Suspendieren der Mikrokapseln, die dem Surfactant ausgesetzt waren, in einem pharmazeutisch verträglichen, mit Wasser nichtmischbaren Öl und Emulgieren der so erhaltenen Suspension in einem wäßrigen Medium.

## Patentansprüche (Patentansprüche für folgende(n) Vertragsstaat(en): ES, GR)

1. Verfahren zur Herstellung einer zur Inhalation geeigneten pharmazeutischen Formulierung, umfassend:
(i) Mikrokapseln, die einen mittleren Durchmesser von 0,1 bis 10 µm aufweisen und die im wesentlichen aus einem biologisch kompatiblen, biologisch abbaubaren polymeren Wandmaterial bestehen, das einen Arzneistoff einkapselt und
(ii) ein lipidlösliches Surfactant, das mit den Mikrokapseln gemischt oder in das Wandmaterial der Mikrokapseln eingebaut ist oder dieses überzieht, wobei das Verfahren das Mischen der Mikrokapseln mit dem Surfactant umfaßt.

2. Verfahren nach Anspruch 1, wobei die Formulierung in Form eines Aerosols zur Inhalation vorliegt.

3. Verfahren nach Anspruch 2, wobei die Konzentration des Surfactants in dem Aerosolbehälter von 0,01 bis 1 Gew.-% beträgt.

4. Verfahren nach Anspruch 1, wobei die Formulierung in Form eines trockenen Pulvers zur Inhalation vorliegt und wobei von 1 bis 10 Gew.-% des Surfactants in die Wände der Mikrokapseln eingebaut sind.

5. Verfahren zur Herstellung einer zum Einnehmen geeigneten pharmazeutischen Formulierung, umfassend:
(i) Mikrokapseln, die einen mittleren Durchmesser von 0,1 bis 20 µm aufweisen und die im wesentlichen aus einem biologisch kompatiblen, polymeren Wandmaterial bestehen, das einen Arzneistoff einkapselt und
(ii) ein lipidlösliches Surfactant, das mit den Mikrokapseln gemischt oder in das Wandmaterial der Mikrokapseln eingebaut ist oder dieses überzieht, wobei das Verfahren das Mischen der Mikrokapseln mit dem Surfactant umfaßt.

6. Verfahren nach einem der Ansprüche 1 bis 5, wobei das Verfahren umfaßt, daß man die Mikrokapseln, die den Arzneistoff einkapseln, einem Surfactant aussetzt, sodaß das Surfactant das Wandmaterial der Mikrokapseln überzieht.

7. Verfahren nach einem der Ansprüche 1 bis 6, wobei das Surfactant in das Wandmaterial der Mikrokapseln eingebaut ist, wobei das Verfahren das Auflösen des polymeren Materials für die Wände in einem Solvens, das Dispergieren des Surfactants und des Arzneistoffs in der Polymerlösung, das Verdampfen des Solvens und die Gewinnung der so gebildeten Mikrokapseln umfaßt.

8. Verfahren nach einem der Ansprüche 5 bis 7, wobei die Formulierung in Form einer zur oralen Verabreichung geeigneten Zusammensetzung vorliegt, wobei das Verfahren das Suspendieren der Mikrokapseln umfaßt, die dem Surfactant in einem pharmazeutisch verträglichen, mit Wasser nichtmischbaren Öl ausgesetzt wurden, und Emulgieren der so erhaltenen Suspension in einem wäßrigen Medium.

9. Verfahren nach einem der Ansprüche 1 bis 8, wobei das Surfactant ein Sorbitanfettsäureester ist.

10. Verfahren nach Anspruch 9, wobei das Surfactant Sorbitantrioleat ist.

11. Verfahren nach einem der Ansprüche 1 bis 10, wobei das Arzneistoff ein Antibiotikum, ein kardiovaskulärer Arzneistoff, ein Arzneistoff für eine Zubereitung gegen Husten oder Erkältung, ein Peptidarzneistoff, ein Antivirusmittel, ein Antikonvulsivum oder ein chemotherapeutisches Mittel zur Behandlung von Krebs ist.

12. Verfahren nach einem der Ansprüche 1 bis 10, wobei der Arzneistoff ein Bronchodilatator oder ein anderes Antiasthmatikum ausgewählt aus Corticosteroiden, Dinatriumcromoglykat und Antihistaminika ist.

13. Verfahren nach Anspruch 12, wobei der Bronchodilatator ein beta-adrenerger Agonist, ein Xanthin, ein anticholinerges Mittel, ein Calciumantagonist oder ein Leukotrien oder ein Derivat davon ist.

14. Verfahren nach Anspruch 13, wobei das Arzneistoff Salbutamol oder Terbutalin ist.

15. Verfahren nach einem der Ansprüche 1 bis 10 und 12 bis 14, das das Mischen der Mikrokapseln umfaßt, die einen Bronchodilatator einkapseln, mit einem Sorbitanfettsäureester in einer Menge bis zu dem Gewicht der Mikrokapseln.

## Revendications (Revendications pour l'(les) Etat(s) contractant(s) suivant(s): AT, BE, CH, DE, FR, GB, IT, LI, LU, NL, SE)

1. Formulation pharmaceutique appropriée pour l'inhalation, comprenant :
(i) des microcapsules ayant un diamètre moyen de 0,1 à 10 µm, qui sont essentiellement constituées en un matériau de paroi polymèrique biodégradable et biocompatible encapsulant un médicament, et
(ii) un tensioactif lipo-soluble qui est mélangé aux microcapsules ou qui est incorporé dans le matériau de paroi des microcapsules ou qui enrobe lesdites microcapsules.

2. Formulation selon la revendication 1, qui est sous la forme d'un aérosol pour inhalation.

3. Formulation selon la revendication 2, dans laquelle la concentration du tensioactif dans le récipient d'aérosol est de 0,01 à 1 % en poids.

4. Formulation selon la revendication 1, qui est sous la forme d'une poudre sèche pour inhalation et dans laquelle 1 à 10 % en poids de tensioactif sont incorporés dans la paroi des microcapsules.

5. Formulation pharmaceutique ingérable, comprenant :
(i') des microcapsules ayant un diamètre moyen de 0,1 à 20 µm, qui sont essentiellement constituées en un matériau de paroi polymèrique biocompatible encapsulant médicament, et
(ii) un tensioactif lipo-soluble qui est mélangé aux microcapsules ou qui est incorporé dans le matériau de paroi des microcapsules ou qui enrobe les dites microcapsules.

6. Formulation selon la revendication 5, qui est sous la forme d'une composition appropriée pour l'administration par voie orale, dans laquelle les microcapsules, qui sont enrobées par le tensioactif, sont mises en suspension dans une huile non miscible à l'eau, pharmaceutiquement acceptable, qui ensuite, est émulsifiée en milieu aqueux.

7. Formulation selon l'une quelconque des revendications 1 à 6, dans laquelle le tensioactif est un ester d'acide gras de sorbitan.

8. Formulation selon la revendication 7, dans laquelle le tensioactif est du trioléate de sorbitan.

9. Formulation selon l'une quelconque des revendications 1 à 8, dans laquelle le médicament est un antibiotique, un médicament cardiovasculaire, un médicament contre la toux ou une préparation contre le rhume, un médicament peptidique, un agent anti-viral, un agent anti-convulsivant ou un agent chimiothérapique de traitement du cancer.

10. Formulation selon l'une quelconque des revendications 1 à 8, dans laquelle le médicament est un agent bronchodilatateur ou un autre médicament contre l'asthme, choisi parmi les corticostéroïdes, le cromoglycate disodique et les antihistaminiques.

11. Formulation selon la revendication 10, dans laquelle l'agent bronchodilatateur est un agoniste béta-adrénergique, une xanthine, un agent anti-cholinergique, un antagoniste calcique ou un leucotriène ou un de ses dérivés.

12. Formation selon la revendication 11, dans laquelle le médicament est le salbutamol ou la terbutaline.

13. Formulation selon l'une quelconque des revendications 1 à 8 et 10 à 12, dans laquelle les microcapsules, encapsulant un agent bronchodilatateur, sont mélangées à un ester d'acide gras de sorbitan qui est présent en une proportion pouvant atteindre le poids des microcapsules.

14. Procédé pour la préparation d'une formulation pharmaceutique telle que définie selon l'une quelconque des revendications 1 à 13, ledit procédé consistant à mélanger les microcapsules avec le tensioactif.

15. Procédé selon la revendication 14, consistant à mettre en contact les microcapsules, encapsulant le médicament, avec un tensioactif de façon à ce que le matériau de paroi des microcapsules soit revêtu du tensioactif.

16. Procédé de préparation d'une formulation pharmaceutique selon l'une quelconque des revendications 1 à 13, dans lequel le tensioactif est incorporé dans le matériau de paroi des microcapsules, redit procédé consistant à dissoudre le matériau polymère destiné à former les parois dans un solvant, à disperser le tensioactif et le médicament dans la solution de polymère, à évaporer le solvant et à obtenir les microcapsules ainsi formées avec un diamètre moyen de 0,1 à 10 µm.

17. Procédé selon la revendication 16, pour la préparation d'une formulation phamaceutique selon la revendication 5, consistant en outre à mettre en suspension les microcapsules, qui ont été mises en contact avec le tensioactif, dans une huile non miscible à l'eau, pharmaceutiquement acceptable, et à émulsifier la suspension obtenue en milieu aqueux.

## Revendications (Revendications pour l'(les) Etat(s) contractant(s) suivant(s): ES, GR)

1. Procédé pour la préparation d'une formulation pharmaceutique appropriée pour l'inhalation, comprenant :
(i) des microcapsules ayant un diamètre moyen de 0,1 à 10 µm, qui sont essentiellement constituées d'un matériau de paroi polymèrique biodégradable et biocompatible encapsulant un médicament, et
(ii) un tensioactif lipo-soluble qui est mélangé aux microcapsules ou qui est incorporé dans le matériau de paroi des microcapsules ou qui enrobe lesdites microcapsules ; le procédé consistant à mélanger les microcapsules avec le tensioactif.

2. Procédé selon la revendication 1, qui est sous la forme d'un aérosol pour inhalation.

3. Procédé selon la revendication 2, dans laquelle la concentration du tensioactif dans le récipient d'aérosol est de 0,01 à 1 % en poids.

4. Procédé selon la revendication 1, qui est sous la forme d'une poudre sèche pour inhalation et dans laquelle 1 à 10 % en poids de tensioactif sont incorporés dans la paroi des microcapsules.

5. Procédé pour la préparation d'une formulation pharmaceutique ingérable, comprenant :
(i') des microcapsules ayant un diamètre moyen de 0,1 à 20 µm, qui sont essentiellement constituées en un matériau de paroi polymèrique biocompatible encapsulant un médicament, et
(ii) un tensioactif lipo-soluble qui est mélangé aux microcapsules ou qui est incorporé dans le matériau de paroi des microcapsules ou qui enrobe lesdites microcapsules ; le procédé consistant à mélanger les microcapsules avec le tensioactif.

6. Procédé selon l'une quelconque des revendications de 1 à 5, ledit procédé consistant à mettre en contact les microcapsules, dans lesquelles est encapsulé le médicament, avec un tensioactif de façon que le tensioactif forme un revêtement sur le matériau de paroi des microcapsules.

7. Procédé selon l'une quelconque des revendications 1 à 6, dans lequel le tensioactif est incorporé dans le matériau de paroi des microcapsules, ledit procédé consistant à dissoudre le matériau polymère destiné à former les parois dans un solvant, à disperser le tensioactif et le médicament dans la solution de polymère, à évaporer le solvant et à obtenir les microcapsules ainsi formées.

8. Procédé selon l'une quelconque des revendications 5 à 7, dans lequel la formulation est sous la forme d'une composition appropriée à l'administration par voie orale, ledit procédé consistant à mettre en suspension les microcapsules, qui ont été mises en contact avec le tensioactif, dans une huile non miscible à l'eau, pharmaceutiquement acceptable, et à émulsifier la suspension obtenue en un milieu aqueux.

9. Procédé selon l'une quelconque des revendications 1 à 8, dans lequel le tensioactif est un ester d'acide gras de sorbitan.

10. Procédé selon la revendicaiton 7, dans lequel le tensioactif est du trioléate de sorbitan.

11. Procédé selon l'une quelconque des revendications 1 à 10, dans lequel le médicament est un antibiotique, un médicament cardiovasculaire, un médicament contre la toux ou une préparation contre le rhume, un médicament peptidique, un agent anti-viral, un agent anti-convulsivant ou un agent chimiothérapique de traitement du cancer.

12. Procédé selon l'une quelconque des revendications 1 à 10, dans lequel le médicament est un agent bronchodilatateur ou un autre médicament contre l'asthme, choisi parmi les corticostéroïdes, le cromoglycate disodique et les antihistaminiques.

13. Procédé selon la revendication 12, dans lequel l'agent bronchodilatateur est un agoniste béta-adrénergique, une xanthine, un agent anti-cholinergique, un antagoniste clacique ou un leucotriène ou un de ses dérivés.

14. Procédé selon la revendication 13, dans lequel le médicament est le salbutamol ou la terbutaline.

15. Procédé selon l'une quelconque des revendications 1 à 10 et 12 à 14, consistant à mélanger les microcapsules, encapsulant un agent bronchodilatateur, à un ester d'acide gras de sorbitan qui est présent en une proportion pouvant atteindre le poids des microcapsules.
